# EUROPEAN PATENT SPECIFICATION

(11) **EP 3 019 094 B1**
(45) Date of publication and mention of the grant of the patent: **20.11.2019**
(21) Application number: 14822194.8
(22) Date of filing: 09.07.2014
(51) Int. Cl.: A61B 17/08, A61L 27/18, A61L 27/34, A61L 27/36, A61L 31/06, A61L 31/10

(54) **BIODEGRADABLE ARTICLES AND THEIR USE FOR TREATING PELVIC FLOOR DISORDERS INCLUDING EXTRACELLULAR MATRIX MATERIAL**
BIOLOGISCH ABBAUBARE GEGENSTÄNDE UND DEREN VERWENDING BEI DER BEHANDLUNG VON ERKRANKUNGEN DES BECKENBODENS MITHILFE EINES EXTRAZELLULÄREN MATRIXMATERIALS
ARTICLES BIODÉGRADABLES ET LEUR UTILISATION POUR LE TRAITEMENT DE TROUBLES DU PLANCHER PELVIEN, COMPRENANT UNE MATIÈRE DE MATRICE EXTRACELLULAIRE

(30) Priority: 09.07.2013 US 201361844282 P
(43) Date of publication of application: 18.05.2016
(73) Proprietor: AMS Research, LLC, Minnetonka, MN 55343 (US)
(72) Inventor: WATSCHKE, Brian P., Minnetonka, Minnesota 55343 (US)
(74) Representative: Pfenning, Meinig & Partner mbB
(86) International application number: PCT/US2014/045933
(87) International publication number: WO 2015/006436

(56) References cited:
- WO-A1-2011/003422
- WO-A1-2012/014205
- WO-A1-2012/094611
- WO-A2-2005/009498
- WO-A2-2007/100459
- WO-A2-2008/107484
- WO-A2-2013/056049
- US-A1- 2003 023 316
- US-A1- 2010 087 839
- US-A1- 2011 264 190
- US-A1- 2012 052 040

## Description

### FIELD OF THE INVENTION

The present invention relates generally to implantable medical articles, such as meshes and stents, for the treatment of pelvic floor disorders, where the articles are associated with extracellular matrix material. The present invention relates to an implant configured for treatment of a pelvic disorder, comprising a biodegradable polymeric mesh structure which has a plurality of interwoven fibers and a plurality of extracellular matrix (ECM) particles disposed within at least one of said fibers, and wherein the at least one fiber has a coating of ECM particles, as defined in the claims. The invention also relates to a biodegradable urethral stent comprising said implant.

### FIELD OF THE INVENTION

Pelvic health is a medical area of increasing importance, at least in part due to an aging population. Examples of common pelvic ailments include incontinence (*e.g.,* fecal and urinary), pelvic tissue prolapse (*e.g.,* female vaginal prolapse), and other conditions of the pelvic floor.

Urinary incontinence can further be classified as including different types, such as stress urinary incontinence (SUI), urge urinary incontinence, mixed urinary incontinence, among others. Other pelvic floor disorders include cystocele, rectocele, enterocele, and prolapse such as anal, uterine and vaginal vault prolapse. A cystocele is a hernia of the bladder, usually into the vagina and introitus. Pelvic disorders such as these can result from weakness or damage to normal pelvic support systems.

Urinary incontinence can be characterized by the loss or diminution in the ability to maintain the urethral sphincter closed as the bladder fills with urine. Stress urinary incontinence (SUI) generally occurs when the patient is physically stressed.

In its severest forms, vaginal vault prolapse can result in the distension of the vaginal apex outside of the vagina. An enterocele is a vaginal hernia in which the peritoneal sac containing a portion of the small bowel extends into the rectovaginal space. Vaginal vault prolapse and enterocele represent challenging forms of pelvic disorders for surgeons. These procedures often involve lengthy surgical procedure times.

Current repair of various pelvic disorders, such as female incontinence and prolapse repair, typically use a mesh implant made of an inert material such as poly(propylene). Traditional mesh implant repairs can be effective, but often not satisfactory for all patients. Porcine tissue has been used as an alternative to inert materials, such as for sling preparation and repair, but the long term efficacy is not as good. In some methods like prolapse repair, excess tissue can be excised and then the tissues plicated together. However, since this tissue is already compromised there is an increased chance it will fail again in the future, requiring addition repeat procedures. In these cases treatment relies on either compromised tissue to act as fully functional tissue, or using an artificial implant to restore anatomy as well as the tissues' response to these devices. Other pelvic treatments such as TURP for BPH, or TURBT for bladder cancer, can lead to erectile dysfunction from nerve damage in the case of BPH, and issues with the bladder wall in cases of bladder cancer. Other diseases such as OAB (over active bladder) and ICS (interstitial cystitis) have been attributed to 'faulty' nerve signaling causing either overactivity of the bladder in OAB or pain in ICS.

Document WO 2011/003422 A1 relates to reinforced biodegradable scaffolds for soft tissue regeneration, as well as methods for support and for augmentation and regeneration of living tissue, wherein a reinforced biodegradable scaffold is used for the treatment of indications, where increased strength and stability is required besides the need for regeneration of living tissue within a patient. WO 2011/003422 A1 further relates to the use of scaffolds together with cells or tissue explants for soft tissue regeneration, such as in the treatment of a medical prolapse, such as rectal or pelvic organ prolapse, or hernia.

Document US 2011/0264190 A1 provides devices comprising a stent; and a coating on said stent comprising a polymer and an active agent, wherein the active agent comprises at least one of: extracellular matrix and an extracellular matrix component. Methods of preparing a device comprising a stent and a coating on said stent are also provided; said methods comprising: providing a stent; and forming a plurality of layers on said stent; wherein the coating comprises a polymer and at least one of said layers comprises one or more active agents; wherein at least a portion of the active agent comprises at least one of extracellular matrix and an extracellular matrix component. Document WO 2008/107484 A1 relates to a temporary composite scaffold comprising discrete ECM particles formed as a fistula plug. According to WO 2008/107484 A1, when using scaffolds containing ECM material, higher concentrations of ECM surprisingly do not give better cell morphology. Concentrations lower than 60%(w/w) are sufficient to obtain the best cell morphology and distribution.

### SUMMARY OF THE INVENTION

The present invention is defined by the claims. Any subject-matter falling outside the scope of the claims if provided for information purposes only. Any references in the description to methods of treatment refer to the implants of the present invention for use in a method for treatment of the human (or animal) body by therapy.

The present disclosure describes implantable medical articles for the treatment of treating pelvic conditions. Embodiments of the disclosure include implantable medical articles configured for treatment of a pelvic floor disorder or disease, the articles including a biodegradable material, and an extracellular matrix preparation. The articles can be used for the treatment of conditions such as incontinence (various forms such as fecal incontinence, stress urinary incontinence, urge incontinence, mixed incontinence, etc.), vaginal prolapse (including various forms such as enterocele, cystocele, rectocele, apical or vault prolapse, uterine descent, etc.), and other conditions caused by muscle and ligament weakness.

In methods of treatment, the implants can initially provide structural support to tissue in the area of the pelvis. ECM material can be made available to the tissue following implantation, initially and/or following degradation of the biodegradable material. The ECM material can promote tissue regeneration, and enhance the formation of natural tissue structures that provide long term benefit to the patient. For example, use of an absorbable implant initially allows for the pelvic organs to be returned to their proper location or ensure that the ECM is in contact with the proper surrounding tissues while tissue regeneration promoted by the ECM occurs. The initial phases of restoration provide by the implant can be followed by natural restoration, both anatomically and histologically.

The invention provides an implant configured for treatment of a pelvic disorder, the implant comprising a biodegradable polymeric mesh structure, and an extracellular matrix preparation associated with the mesh structure, as defined in the claims.

After implantation the implant can provide tissue support, degrade over a period of time, and deposit the ECM material in the implantation area for regeneration of tissue and long term benefit.

In another embodiment, the invention provides an implant or stent as defined in the claims configured for treatment of a pelvic disorder, the implant formed from material comprising an extrusion comprising a biostable or biodegradable polymeric material and extracellular matrix preparation.

Further, the invention provides a biodegradable urethral stent comprising a biodegradable polymer and an extracellular matrix preparation as defined in the claims. Moreover, the invention provides a penile implant comprising an extracellular matrix preparation as defined in the claims.

The implantable medical articles of the invention can be used for treatment of a pelvic floor condition. Uses can include steps of (a) providing an medical implant comprising an ECM preparation as described herein, and (b) implanting the implant at a target site in the pelvic area to treat the pelvic floor condition. The pelvic floor condition can be an incontinence

condition, such as one selected from the group consisting of fecal incontinence, stress urinary incontinence, urge incontinence, mixed incontinence, a vaginal prolapse condition, such as one selected from the group consisting of enterocele, cystocele, rectocele, apical or vault prolapse, and uterine descent, a male urogenital condition such as erectile dysfunction and benign prostatic hypertrophy (BPH), a bladder conditions such as interstitial cystitis (IC), overactive bladder (OAB), and bladder cancer, or other conditions caused by muscle and ligament weakness.

Use of the ECM preparation can promote regeneration of various tissue types include muscle tissue and tendon, nervous tissue, connective tissue, and epithelial tissue.

Yet other aspects of the disclosure are directed towards kits or systems for the treatment of a pelvic floor condition. The kits or systems include (a) an implantable medical article comprising an ECM preparation as described herein, and (b) one or more instruments for the insertion of the article in a subject.

### BRIEF DESCRIPTION OF THE DRAWINGS

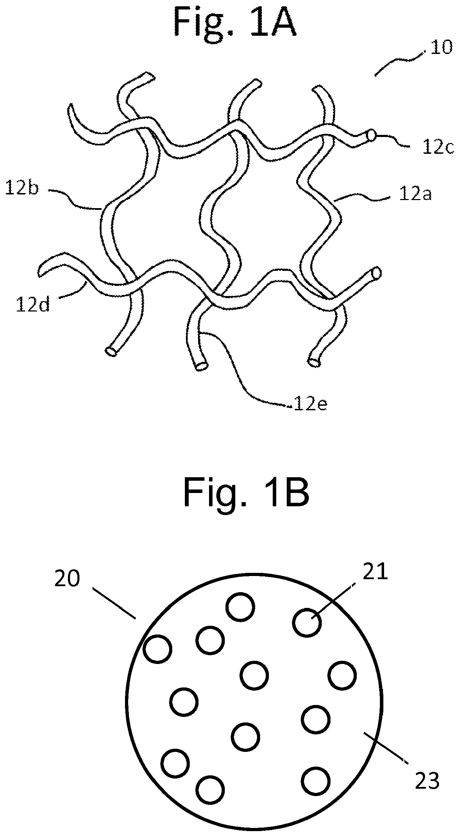

Figure 1a is an illustration of a portion of a biodegradable mesh with biodegradable monofilaments associated with an ECM preparation.
Figure 1a is an illustration of cross section a biodegradable monofilament with ECM particles located therein.
Figure 2a is an illustration of a portion of a biodegradable mesh having an ECM coating.
Figure 2b is an illustration of a cross-section of a biodegradable monofilament having an ECM coating on the surface of the monofilament.
Figure 2c is an illustration of a cross-section of a biodegradable monofilament having an ECM particle coating on the surface of the monofilament.
Figure 2d is an illustration of a cross-section of a biodegradable monofilament having an ECM particle and biodegradable polymer coating on the surface of the monofilament.
Figure 3 (reference) is an illustration of a portion of a biodegradable molded mesh associated with an ECM preparation.
Figure 4 is an illustration of a biodegradable urethral stent including braided monofilaments associated with an ECM preparation.
Figure 5 (reference) is an illustration of a molded or extruded biodegradable urethral stent including fenestrations, the stent associated with an ECM preparation.

### DETAILED DESCRIPTION

Pelvic implants or devices can be associated with an extracellular matrix (ECM) preparation. As a general matter, an ECM preparation refers to a processed preparation of a tissue that contains a mixture of structural and functional molecules secreted by the resident cells of the tissue from which the ECM preparation is prepared. The particular ECM constituents and their abundance in the preparation can vary depending on the tissue type that is processed, as well as the processing parameters. The ECM preparation can be prepared by obtaining a desired tissue, removing cellular material from the tissue, and then processing the decellularized tissue to a desirable form. Processing can include steps of dehydration or lyophilization, and disinfection. Exemplary tissue sources include small intestinal submucosa (SIS), urinary bladder, cardiac tissue, vasculature, dermal tissue, nervous tissue, muscle, tendons, ligaments, and liver. Tissue can be obtained from syngenic, allogenic, or xenogenic sources.

Processing steps can remove antigenic components, such as those associated with cell membranes and intracellular components. This generally minimizes or eliminates host immunologic responses when the implant with ECM material is introduced into a patient for the treatment of a pelvic condition. Decellularization of ECM preparations can include chemical and/or physical processes. For example, processed tissue can be subjected to processing steps such as freezing and thawing, sonication, chopping, mincing, etc. Detergents, such as anionic detergents and non-ionic detergents, can be used for decellularization. Examples of anionic detergents are sulfate-based detergents such as sodium sodium dodecyl sulfate (SDS), and examples non-ionic detergents are poly(alkylene oxide)-based detergents such as Triton X-100. Enzymes such as proteases, like trypsin, can also be used for processing the tissue.

Most ECM preparations have collagen as the primary solids component in the preparation (> 50% wt), and more typically greater than 75% wt, or greater than 90% wt. Most of the collagen of the collagen component is collagen type I. Other collagen types such as collagen type III, IV, V, VI and VII can be present in the preparation in smaller amounts. ECM preparations can also include glycosaminoglycans, such as hyaluronic acid, chondroitin sulfate, heparin, and heparan sulfate. ECM preparations can also include adhesion molecules such as fibronectin and laminin, as well as proteoglycans and glycoproteins. Growth factors such as vascular endothelial growth factor (VEGF) transforming growth factor-b (TGF-β), and basic fibroblast growth factor (β-FGF) can also be present in ECM preparations. ECM proteins are understood to have high conservation across species and therefore are generally immunologically tolerated in xenogeneic recipients.

ECM preparations are commercially available from a variety of manufacturers (e.g., Bard, Synovis, Cook, Lifecell, etc.) and can be provided in a variety of forms such as a dry sheet, a hydrated sheet, or a gel.

In some modes of practice, an ECM powder is obtained or prepared and associated with the pelvic implant. For example, the ECM powder can be prepared according to the process described by Gilbert et al. (2005) Biomaterials 26:1431-1435, in which methods to produce a particle form of extracellular matrix (ECM) from porcine urinary bladder are described.

In one method of preparing ECM powder, a lyophilized form of ECM material is snap frozen, and then pulverized in a grinding mill. In another method of preparing ECM powder, a lyophilized form of ECM material is saturated in a NaCl composition, snap frozen to precipitate salt prior to grinding, and then pulverized in a grinding mill.

ECM can be obtained from porcine urinary bladder by removing the tunica muscularis externa and tunica submucosa layers, leaving the basement membrane and tunica propria intact. The urinary bladder matrix (UBM) can then be washed in a 0.1% peracetic acid solution for two hours with subsequent rinses in phosphate buffered saline and distilled water to disinfect the material and remove any cellular remnants.

In one mode of practice the UBM is made into particle form by lyophilizing disinfected UBM material and then chopping it into small sheets for immersion in liquid nitrogen. Snap frozen UBM is then reduced to small pieces with a Waring blender to make particles sized to fit in a rotary knife mill. A #60 screen is used to restrict the collected powder size to less than 250 µm.

In another mode of practice the UBM is made into particle form by first soaking the disinfected UBM material in a 30% (w/v) NaCl solution for 5 min. The NaCl-soaked UBM is then snap frozen in liquid nitrogen to precipitate salt crystals, and next lyophilized to remove residual water. This material is then comminuted according to the first mode of preparation. Precipitating NaCl is to promote the UBM material to fracture into more uniformly sized particles. The particles are suspended in deionized water and centrifuged (5 min at 1000 rpm, three times) to remove NaCl. The suspension is snap frozen, lyophilized, and then the powder placed in a rotary knife mill to disaggregate the individual particles.

Exemplary sizes of ECM particles can be in the micron range (e.g., 1 µm to 1000 µm, 5 µm to 750 µm, or 10 µm to 500 µm), with mean particle sizes in the range of about 50 µm to about 200 µm. Populations of larger or smaller particles can be generated by sifting using screens with openings of predetermined sizes. The particles can have irregular shapes with sheet-like or fiber-like appearances. The particles can also have different textured surfaces (such as a smooth surface and a more fibrous surface).

Various types of absorbable polymeric materials can be used to form the implantable medical article, such as a mesh, or a prosthetic device like a stent. The terms "bioabsorbable," "degradable," and "biodegradable," can also be used to describe a material that is absorbable, such as an absorbable polymer. Many absorbable polymers include hydrolytically unstable chemical groups such as ester groups in the polymeric backbone. The hydrolytic cleavage of these chemical groups leads to degradation of the polymer.

Synthetic bioabsorbable polymers include polyhydroxyalkanoates (e.g., poly-4-hydroxybutyrate (P4HB), poly(3-hydroxyvalerate), and poly(hydroxybutyrate-co-hydroxyvalerate)); polyesters (e.g., polylactic acid, poly(lactide-co-glycolide), polycaprolactone, poly(valerolactone), poly(glycolic acid), (poly(glycolide)), and poly(dioxanone)); polyorthoesters; polyalkeneanhydrides (e.g., poly(sebacic acid)); polyanhydrides, and polyphosphazine.

Polyhydroxyalkanoates include homopolymers such as poly-4-hydroxybutyrate (P4HB), poly(3-hydroxyvalerate), and hydroxyalkanoate copolymers such as poly(hydroxybutyrate-*co*-hydroxyvalerate) (Organ, S.J. (1994) Polymer, 35, 1:86-92). Blends of hydroxyalkanoate polymers with other absorbable polymers have also been prepared, such as poly(β-hydroxybutyrate) and poly(ε-caprolactone) blends (Gassner, F., and Owen, A.J. (1994) Polymer, 35, 10:2233-2236).

Poly(glycolic acid) (PGA) is a highly crystalline and has a melting point in the range of 225-230°C. While higher molecular weight forms are insoluble in common organic solvents such as acetone, dicholomethane, chloroform, and tetrahydrofuran, its lower molecular weight forms generally have better solubility in common organic solvents. Glycolide copolymers also can have better solubility in common organic solvents. For example, star block copolymers based on glycerol and glycolide show solubility in organic solvents such as DMF and DMSO (see, for example, Wolf, F.K., et al. (2010) Beilstein J. Org. Chem. 6, No. 67). Copolymers of lactic acid and glycolic acid (e.g., 50:50 mol percent) have solubility in chloroform (United States Patent 3867190). Copolymerization of lactic acid and glycolic acid reduces the degree of crystallinity and results in an increased rate of hydration and hydrolysis. Copolymers of lactic acid and glycolic acid can be manipulated into a desired form by techniques such as extrusion, injection and compression molding as well as particulate leaching and solvent casting.

Lactic acid is a chiral molecule and L-lactide and D-lactide optically active forms can be polymerized to form poly-L-lactide (PLLA), poly-D-lactide (PDLA), and poly-D,L-lactide (PDLLA). PLLA has a crystallinity of about 37%, a glass transition temperature between 60-65°C, and a melting temperature between 173-178 °C. PDLLA is amorphous and has a glass transition temperature of 55-60°C.

Another polyester, polydioxanone (PDS) is made by a ring-opening polymerization of the *p*-dioxanone monomer that forms a polymer of multiple repeating ether-ester units. PDS has a glass transition temperature in the range of - 10 to 0°C and a degree of crystallinity of about 55%. The presence of an ether oxygen within the polydioxanone backbone of the polymer chain can provide materials with enhanced flexibility.

Exemplary erodible polyorthoesters can be formed by reacting an orthoester (or orthocarbonate) with a diol (see, for example, U.S. Pat. Nos. 4,079,038, and 4,138,344), or by reacting a reacting a polyol with a polyfunctional ketene acetal (see, for example, U.S. Pat. No. 4,304,767).

The implant may also include a natural polymer that can be absorbed in the body. Hyaluronic acid, alginate, dextran, starch, amylopectin, cellulose, xanthan, pullulan, chitosan, pectin, inulin, and heparin are examples of absorbable natural polymers which can be used to prepare the implantable medical article.

The implant can also include biodegradable material that can be cured by light irradiation. For example, a composition that includes a light-curable biodegradable polymer and ECM material can be irradiated, which causes the composition to solidify, such as by formation or crosslinking of polymeric material. The composition may be in the placed in a mold and irradiated, or the solidified composition can be used to construct the implant. Photosensitive reagents that can be used to cause polymerization of a composition to form the implant are commercially available (for example, Irgacure™, BASF). Exemplary photosensitive compounds initiate free radical polymerization of polymerizable material in the composition following UV exposure. Exemplary polymerizable materials include monomers and polymers (macromers) with unsaturated groups. For example, the composition can include a divinyl terminated poly(lactide-co-caprolactone) macromers which can be crosslinked in the presence of a light initiating system (see, for example, Davis, K.A. (2003) Biomaterials 24:2485-95).

In embodiments, some pelvic implants of the disclosure do not include any, or any substantial amount, of an inert material or a non-degradable material. For example, in some embodiments, the implant does not include any, or any substantial amount, of a non-degradable synthetic polymer, such as poly(propylene).

A biodegradable implant with ECM can include one or more portions made from woven biodegradable materials. For example, the implant can include a central support portion and/or extension portion(s) made from woven materials. The woven portion(s) can be made from monofilaments, multifilaments, yarns of polymeric material, or combinations thereof. A woven mesh generally has openings and the size and shape of these openings can be defined by the weave or knitting patterns of the woven mesh. The openings can be of any one or combination of shapes, such as square, rectangular, triangular, oval, circular, or more complex polygonal shapes (hexagonal, etc.), as well as irregular shapes, such as might be associated with more complex knitted or woven constructs.

In some embodiments the implant portions having a knitted or woven construction include biodegradable monofilaments, such as poly(L-lactide) (PLLA) monofilaments (see, for example, Kinoshita, Y. et al. (1993) Biomaterials; 14:729-36). Exemplary monofilaments have diameters in the range of about 10 µm to about 250 µm (∼ 0.0004 to ∼ 0.01 inches), or more specifically from about 25 µm to about 150 µm (∼ 0.001 to ∼ 0.006 inches). Commercial examples of absorbable materials include Dexon™ (polyglycolic acid) available from Davis and Geck of Danbury, Connecticut, and Vicryl™ available from Ethicon.

In some embodiments, all or a portion of the implant can be made from a woven mesh that includes ECM material. For example, the implant can have a central support portion and one or more extension portions, either of which, or both, can be made from a woven mesh with ECM material. Alternatively, one portion of the implant can be made from a woven mesh, and another portion of the implant can be made from a material that is different than the woven mesh, such as a molded, non-woven, mesh.

The sizing and shape of an implant that includes a woven filament material can provide the structure as well as the pore size to provide the proper amount of surface area to deliver the ECM to the treatment site. Exemplary sizes of apertures in a mesh construct can be in the range of about 0.2 mm² to about 2 mm², or more specifically in the range of about 0.5 mm² to about 1.5 mm².

In some cases the mesh can also be defined in terms of its basis weight. In many constructions a mesh with a lower basis weight can be more porous or have larger openings, whereas a mesh with a higher basis weight is less porous or has smaller openings. In some aspects the mesh has a basis weight in the range of about 5 g/m² to about 100 g/m², and more specifically in the range of about 10 g/m² to about 50 g/m², or about 15 g/m² to about 30 g/m².

Mesh porosity can also be expressed in terms of percent porosity. "Porosity" refers to the percentage of the mesh surface that has openings. In exemplary constructions, the mesh preferably has porosity of greater than 50%, and more preferably greater than 60%, 70%, or 75%.

In some embodiments, the implant comprises a porous mono- or multifilament, or a molded construction, and the ECM material can be included in the pores of the molded or filament-based material. Porous absorbable mono- or multifilaments can be prepared using a biodegradable polymer, or a mixture thereof, along with a porogen, such a salt, sucrose, PEG, or an alcohol. Removing a porogen from a biodegradable matrix is described in U.S. Patent No. 5,948,020.

As a general matter, a porogen can be dispersed in a composition that includes a biodegradable polymer or a combination of biodegradable polymers. In some cases the porogen/polymer composition is melt processed into a desired form, such as a monofilament or a molded article. The monofilament or molded article can also be formed by dissolving the polymer in a solvent (e.g., an organic solvent) and dispersing the porogen therein, and then casting the article from the composition. After the monofilament or molded is formed, or after the monofilament is formed into a fabric, the porogen can be removed using a solvent that dissolves the porogen out of the polymeric material of the article.

After the pores are created in the implant article, the article can be soaked in a solution that includes the ECM material, such as a preparation of ECM particle material.

In other modes of practice, powderized ECM is impregnated into biodegradable polymeric material of the implant using a process selected from the group consisting of swelling of implant material, and vacuum incorporation.

In one construction according to the invention, as shown in Figure 1a, the biodegradable implant comprises a mesh made from multiple fibers. A portion of the mesh with fibers **12a** - **12e** is shown. One of more of the fibers can include the ECM material, such as ECM particles, within the degradable material of the fibers. The biodegradable material (e.g., a PLLA polymer) can comprise the majority of the amount of the fibers in the mesh, such as greater than 50% (wt), 60% (wt) or greater, 70% (wt) or greater, 80% (wt) or greater, 90% (wt) or greater, 95% (wt) or greater, or 97.5% (wt) or greater. The remaining material in the mesh can be the ECM material.

Figure 1b shows a cross-section of a fiber of the mesh of Figure 1a having ECM particles (e.g., **21**) within the biodegradable material **23** of the fiber. The ECM particles can be surrounded by biodegradable material, or a portion of the particles can be in contact with biodegradable material, or both. The mesh can optionally be described in terms of the relationship between the size of the fiber and the ECM particle (e.g., diameter:diameter). The "diameter" of an ECM particle can be the average cross-section of ECM particles in a preparation, which may include particles of various shapes and sizes. For example, the ratio of the diameter of the ECM particle to the diameter of the mesh can be in the range of about 1:100 to about 1:5, or more specifically about 1:50 to about 1:10.

An implant that has ECM material incorporated into the biodegradable material of the mesh can release the ECM material in the area of implant placement during and/or after the implant degrades. Therefore, the implant can serve as a timed-release mechanism for the ECM material. A biodegradable polymer or polymer combination can be chosen to provide a desired ECM release mechanism.

In other embodiments, the biodegradable structure of the implant is formed by preparing a composition comprising a biodegradable polymer and ECM material, such as ECM particles as described herein. For example, in one mode of preparation, a composition of a biodegradable melt-processable polymer and ECM material is heated and the composition is formed into a filament, such as by extrusion, or introduced into a mold. In another mode of preparation, a composition including a biodegradable melt-processable polymer dissolved in solvent and ECM material dispersed therein is prepared and then the article is casted from the composition.

According to the invention, the biodegradable structure of the implant is coated with a composition comprising an ECM material, such as ECM particles as described herein. For example in one non-claimed mode of preparation, a composition of an ECM material is applied to a surface of the biodegradable article using a process such as brushing, swabbing, or dipcoating of the composition on the article. Depending on the amount or concentration of the ECM material used, the ECM coating formed on the biodegradable article can be contiguous or non-contiguous.

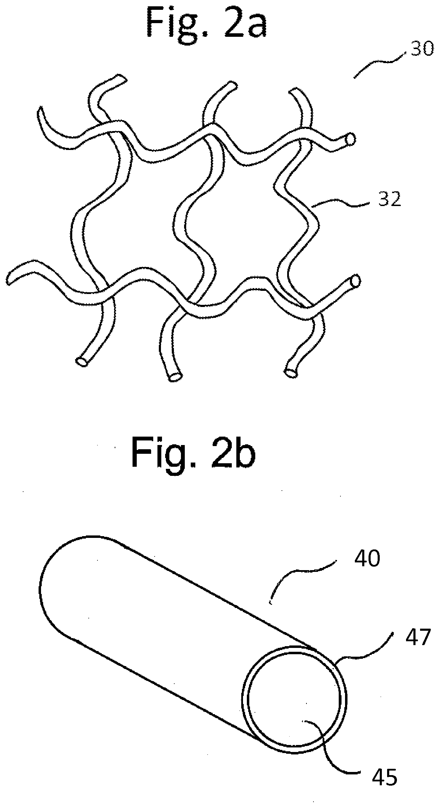

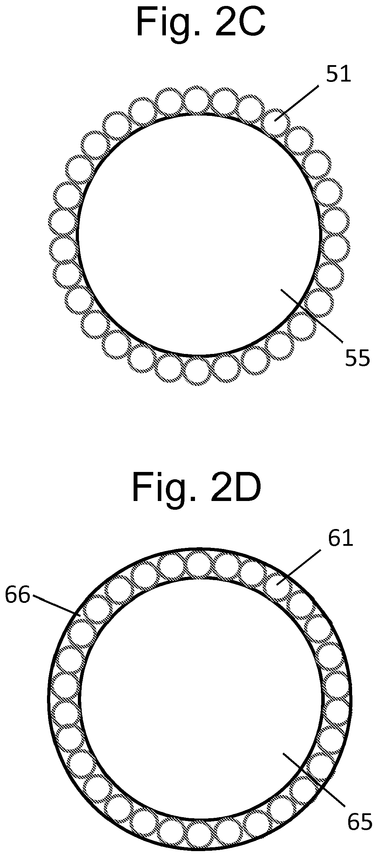

According to the invention, as shown in Figure 2a, the biodegradable implant comprises a mesh **30** made from multiple fibers, with one (e.g., **32**) of more of the fibers having a coating of ECM material, such as ECM particles. Figure 2b shows a cross-section of a fiber of a biodegradable mesh having ECM material as a coating **47** on the outer surface of a biodegradable fiber **45.** Figure 2c shows in detail a cross-section of a fiber of a biodegradable mesh having ECM particles **51** as a coating on the outer surface of a biodegradable fiber **55**. According to the invention, adherence of the ECM particles to the surface of the fiber is enhanced by using a binder (not shown). Figure 2d shows in a cross-section of a fiber of a biodegradable mesh having ECM particles **51** and a biodegradable polymeric material **66** as a coating on the outer surface of a biodegradable fiber **65,**

In some examples, the pelvic implant with ECM material can be formed from a non-knitted/non-woven (e.g., molded) polymeric mesh layer (see, for example, commonly assigned PCT Publication Nos. WO 2011/063412 and WO 2011/072148). Non-knitted/non-woven meshes can be formed of patterned cells by way of a molding, die casting, laser etching, laser cutting, extruding, punching, or 3-D printing process. The portion of the implant that is the non-knitted/non-woven mesh may be considered a homogenous unitary construct. The pattern cut or formed implant can be constructed of a biodegradable polymer material to provide a lattice support structure of repeated apertures or cells. Repeated apertures forming a lattice structure can be cut or molded into sinusoid, or other waveform or undulating strut patterns to control elongation or compression along single or multiple axes to define a desirable pattern density with overall reduced surface area, and to control the distribution and shaping from applied loads.

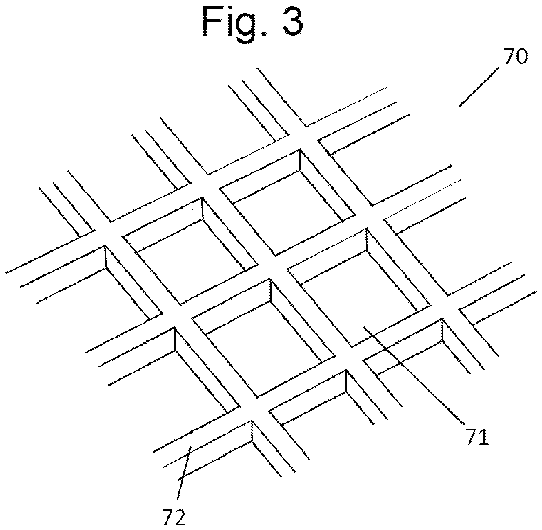

In an exemplary non-claimed construction, as shown in Figure 3, the biodegradable implant comprises a molded mesh **70** including an ECM material. The molded mesh **70** is shown having generally square or rectangular apertures **71,** as formed from struts or crosspieces represented by item **72.** However, a molded biodegradable mesh associated with an ECM material can be formed to have apertures and struts or crosspieces of any desired shape and configuration. ECM material (not shown) can be located within biodegradable material of the struts or crosspieces, or as a coating on all or a portion of the struts or crosspieces.

Figure 2b shows a cross-section of a fiber of a biodegradable mesh having ECM material as a coating **47** on the outer surface of a biodegradable fiber **45.** Figure 2c shows in detail a cross-section of a fiber of a biodegradable mesh having ECM particles **51** as a coating on the outer surface of a biodegradable fiber **55.** Adherence of the ECM particles to the surface of the fiber may be enhanced by using a binder (not shown). Figure 2d shows in a cross-section of a fiber of a biodegradable mesh having ECM particles **51** and a biodegradable polymeric material **66** as a coating on the outer surface of a biodegradable fiber **65,**

The ECM material can be associated with a non-knitted/non-woven mesh in ways as described, such as by (a) coating the surface of the non-knitted/non-woven mesh, (b) using a composition that includes biodegradable polymer and an ECM material for extruding or solvent casting the non-knitted/non-woven mesh, or (c) preparing a porous non-knitted/non-woven mesh using a porogen and then filling the pores with ECM material.

In some examples, all or a portion of the implant is prepared from a composition comprising ECM material and a biocompatible binder, wherein the ECM material is the primary component by weight the composition. The composition can be prepared by mixing ECM material, such as ECM particles, and the biocompatible binder component, optionally in the presence of a solvent. In this embodiment, the ECM material is the primary solids component by weight in the composition. For example, the composition can include ECM material in an amount of 50% (wt.) or greater, 60% (wt.) or greater, 70% (wt.) or greater, 80% (wt.) or greater, 90% (wt.) or greater, or 95% (wt.) or greater.

In some examples the biocompatible binder is selected from the group consisting of proteins, polysaccharides, nucleic acids, carbohydrates, or mixtures thereof. The biocompatible binder can be absorbable in the body. The biocompatible binder can be used in an amount less then the ECM material. For example, the biocompatible binder can be present in an amount of less than 50% (wt.), less than 40% (wt.), less than 30% (wt.), less than 20% (wt.), less than 10% (wt.), or less than 5% (wt.).

In use, the implant can degrade over a period of time, providing ECM material at the site of implant placement. For example, the implant can be placed at a site of tissue repair in the pelvic area to provide support for a period of time. The binder material can degrade during and/or after the time support is provided by the implant. Degradation of the binder can deposit the ECM material in the area of implant placement and promote tissue regeneration and strengthening for long-term repair.

The biodegradable implants with ECM as described herein can be used in association with known implant and repair systems (*e.g*., for male and female), including those disclosed in U.S. Patent Nos. 7,500,945, 7,407,480, 7,351,197, 7,347,812, 7,303,525, 7,025,063, 6,691,711, 6,648,921, and 6,612,977, International Patent Publication Nos. WO 2008/057261 and WO 2007/097994, and U.S. Patent Publication Nos. 2010/0105979, 2002/151762 and 2002/147382.

For example, some embodiments, the implant with ECM material is configured for implantation into a female patient. Portions of the implant can have features to support an anatomical structure in the pelvis (i.e., a "support portion"), such as the vagina, bladder, urethra, or levator ani. Portions of the implant can also have features, such as straps or arms that extend from a support portion of the implant, or tissue anchors or fasteners (e.g., self-fixating tips), to help maintain the implant at a desired anatomical location in the pelvis.

For example, the implant with ECM material can be used for treating urinary incontinence in a female subject, the implant including a urethral sling having a central portion and first and second ends or arms. The first and second ends/arms can be coupled to and extend from the central support portion. Following implantation, the arms are used to help secure or position the implant at a desired anatomical location in the pelvis.

Implants with ECM material of the invention can be part of a kit. The kit can include components for carrying out procedures for the insertion of the implant in a patient. Exemplary components can include tissue fasteners, tools for introducing the implant into a patient using a surgical insertion procedure, scalpels or knives for making the incision, and needles and suture material for closing the incision. All or parts of the kit can be sterilely packaged. Insertion tools useful for insertion of the implant can include a handle and an elongate needle, wire, or rod extending from the handle. The needle, wire, or rod can be shaped (such as helical, straight, or curved) to be useful to carry the implant through a desired tissue path in the pelvic region.

The particular features of the implant embodiments of the invention can be adapted to known implant constructions useful for treating female pelvic conditions, including those already described in the art. Those skilled in the art will recognize that various other mesh configurations, such as those described herein with reference to the following publications, can also be used in conjunction with the features and procedures of the current invention.

In some constructions, the implant with ECM material is used for treating incontinence, prolapse, or a mixture of incontinence and prolapse, and includes a portion useful to support the urethra or bladder neck to address urinary incontinence, such as described in commonly assigned application published as US 2010/0256442 (Ogdahl, et al.), and exemplified by the mesh constructions of Figures 3B and 3C therein. The implant can be in the form of a biodegradable mesh strip that in inserted transvaginally and used to support the urethra or bladder neck. The implant with ECM material can be configured to have a length (distance between distal ends, e.g., self-fixating tips, of extension portions) to extend from a right obturator foramen to a left obturator foramen, (e.g., from one obturator internus muscle to the other obturator internus muscle). Exemplary lengths of an implant or implant portion for extension below the urethra, between opposing obturator foramen, from distal end to distal end of the extensions while laying flat, can be in the range from about 6 to 15 centimeters, e.g., from 7 to 10 centimeters or from 8 to 9 centimeters or about 8.5 centimeters. (Lengths L1 and L2 of figures 3B and 3C can be within these ranges.) The lengths are for female urethral slings, and are for anterior portions of implants for treating female prolapse or combined female prolapse and incontinence, which include an anterior portion that has a length between ends of anterior extensions portions within these same ranges. A width of the extension portion can be as desired, such as within the range from about 1 to 1.5 centimeters. The implant can also have two or more tissue anchoring features (e.g., self-fixating tips). The self-fixating tips can be present at the ends of the mesh strips, or at the ends of arms or extensions that extend from a central support portion.

In some constructions, mesh with ECM material can be configured to treat pelvic conditions by supporting levator muscle, such as described in commonly assigned application published as US 2010/0261952 (Montpetit, et al.). The levator musculature or "levator ani" can include the puborectalis, pubococcygeus, iliococcygeus. Exemplary implants can be of a size and shape to conform to levator tissue, optionally to additionally contact or support other tissue of the pelvic region such as the anal sphincter, rectum, perineal body, etc. The implant can be of a single or multiple pieces that is or are shaped overall to match a portion of the levator, e.g., that is circular, oblong trapezoidal, rectangular, that contains a combination of straight, angled, and arcuate edges, etc. The implant can include attached or separate segments that fit together to extend beside or around pelvic features such as the rectum, anus, vagina, and the like, optionally to attach to the feature. The implant can include a tissue support portion, which at least in part contacts levator tissue. Optionally, the implant can additionally include one or more extension portion(s) that extends beyond the tissue support portion and to be secured to tissue of the pelvic region, for support of the tissue support portion. Optionally, extension portions can include features such as a tissue fastener (e.g., self-fixating tip, soft tissue anchor, bone anchor, etc.), a sheath, a tensioning mechanism such as a suture, an adjustment mechanism, etc.

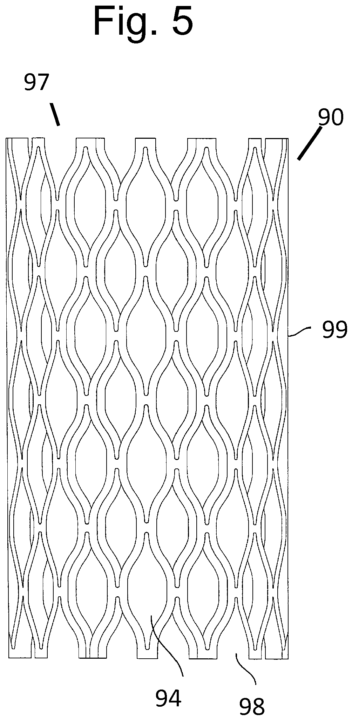

According to exemplary methods, an implant for supporting levator muscle can be introduced through a vaginal incision that allows access to levator tissue. The method can include use of an insertion tool designed to reach through a vaginal incision, through an internal tissue path and to then extend through a second external incision. In some cases a tools is used to place a self-fixating tip at an internal location of the pelvic region, the tool length sufficient to reach from a vaginal incision to an obturator foramen, region of the ischial spine, sacrospinous ligament, or other location of placing a self-fixating tip. Exemplary methods include steps that involve creating a single medial transvaginal incision and dissecting within a plane or region of dissection including the ischorectal fossa. An implant with ECM material can be inserted to contact tissue of the levator, over a desired area. A kit with the implant can include connectors for engagement between a needle of an insertion tool and a distal end of an extension portion, as well as helical, straight, and curved needles. An embodiment of a kit, including an insertion tool and an implant, is shown in Figure 5 of US 2010/0261952.

The implant with ECM material can include self-fixating tips designed to engage a distal end of an insertion tool to allow the insertion tool to place the self-fixating tip at a desired tissue location by pushing. For example, the mesh can be implanted by creating a single medial transvaginal incision under the mid-urethra, dissecting a tissue path on each side of the incision, passing a urinary incontinence sling through the incision whereby the urinary incontinence sling is suspended between the obturator internus muscles and the sling body is positioned between the patient's urethra and vaginal wall to provide support to the urethra. Commonly assigned application published as US 2011/0034759 (Ogdahl, et al.), also describes implants that include a self-fixating tip at a distal end of one or more extension portions, and transvaginal methods for inserting the mesh into a patient.

In some constructions, mesh with ECM material an be configured to treat vaginal prolapse, including anterior prolapse, posterior prolapse, or vault prolapse such as described in commonly assigned application published as US 2010/0261955-A1 (O'Hern, et al.). The mesh can be inserted transvaginally, following a single incision in the vaginal tissue, with no external incision. The mesh can be used to provide Level 1 support of the vaginal apex in combination with Level 2 support of medial vaginal sidewall tissue. In terms of vaginal prolapse, Level 1 vaginal tissue support relates to support of the top portion, or "apex" of the vagina. This section of tissue is naturally supported by the cardinal ligament that goes laterally to the ischial spine and crosses over medially to the sacrospinous ligament, and also by the uterosacral ligament that anchors into the sacrum. Level 2 support of vaginal tissue is support of tissue of the mid section of the vagina, below the bladder. This tissue is partially supported by the cardinal ligament but is predominantly supported by lateral fascial attachments to the arcus tendineus or white line. Level 3 support is that of the front end (sometimes referred to as the "distal" section) of the vagina right under the urethra. Natural support includes lateral fascial attachments that anchor into the obturator internus muscle.

A method for inserting the implant with ECM material for treating vaginal prolapse can include providing an implant that includes a tissue support portion and two or more extension portions; placing the tissue support portion in contact with vaginal tissue to support the vaginal tissue; and extending a posterior extension portion to engage a sacrospinous ligament, and extending a lateral extension portion to engage tissue at a region of ischial spine, or extending a posterior extension portion to engage a sacrospinous ligament, and extending an anterior extension portion to engage an obturator foramen, or extending an extension portion to engage a sacrospinous ligament to provide Level 1 support, and supporting vaginal tissue to provide Level 2 support. Figure 16 of US-2010-0261955-A1 illustrates a kit with an implant having a support portion piece, two extension portion pieces, adjusting tool, grommet management tool, and insertion tool.

In some modes of practice, the implants with ECM material can be used along with an expansion member in a sacral colpopexy is a procedure for providing vaginal vault suspension, such as described in commonly assigned International Application No. PCT/US11/53985. A sacral colpopexy generally involves suspension, such as by use of a mesh strip implant, of the vaginal cuff to a region of sacral anatomy such as the sacrum (bone itself), a nearby sacrospinous ligament, uterosacral ligament, or anterior longitudinal ligament at the sacral promontory. The implant can be utilized in a transvaginal sacral colpopexy (TSCP) procedure with an expansion member to access tissue of the posterior pelvic region.

In some embodiments, the pelvic implant is in the form of a urethral stent. The urethral stent can be formed from biodegradable monofilaments, such as monofilaments that are woven or braided into a desired configuration. In other examples the urethral stent can be formed by extrusion or injection molding to form a biodegradable tubular structure. The tubular structure can include openings (fenestrations) that can be formed during or after the extrusion process. Biodegradable polymers, including those described herein such as polyanhydrides, polycaprolactones, polyglycolic acids, poly-L-lactic acids, poly-D-L-lactic acids, and polyphosphate esters, can be used to form the filaments or tubular structure of the urethral stent.

The ECM material can be associated with monofilaments of the urethral stent in ways as described, such as by coating the filaments or tubular structure, or co-extruding the ECM material with the biodegradable polymer.

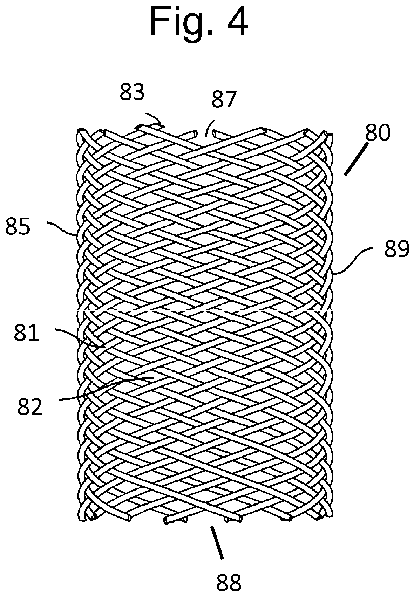

In one embodiment, as shown in Figure 4, the urethral sent includes woven monofilaments formed from one or more biodegradable polymers, and an ECM material that is incorporated into the woven monofilaments, or coated on the surface of the monofilaments. In Figure 4, the stent **80** is a tubular shaped member having first and second ends **87, 88,** a walled surface **89** disposed between the first and second ends. The walls are composed of extruded polymer monofilaments **85** woven into a braid-like configuration. ECM material (not shown) can be incorporated into the biodegradable polymer of the woven monofilaments, or coated on the surface of the monofilaments.

The urethral stent can be made by interweaving the monofilaments **85** in a helical pattern on a round bar mandrel such that one-half of the monofilaments are wound clockwise. Each monofilament intersects **85** the oppositely wound monofilaments in an alternating over-under pattern such that a tubular braid is made with crossing angles **82** between overlapping monofilaments in the longitudinal or axial direction (when the stent 10 is in a non-compressed, resting position) of 100-150 degrees. The braided device is transferred to an annealing mandrel having a diameter equal to or less than the round braiding mandrel The ends **83** of the braid are compressed or extended to yield the optimum post annealing geometry, then the ends are secured to the annealing mandrel The device is then annealed by heating the annealing bar and stent to 90°C for one hour in an inert atmosphere followed by a second heating cycle for 2 hours at 140°C in the same inert atmosphere The stent is not allowed to cool between heating cycles. Finally, the stent is cooled, removed from the annealing bar and cut to the desired length.

As shown in Figure 5 (reference), the urethral stent may include an injection molded or extruded fenestrated tube and an ECM material that is incorporated into the biodegradable material of the tube, or coated on the surface of the tube. In Figure 5, the urethral stent **90** is injection molded or extruded. In Figure 5, the stent **90** is a tubular shaped member having first and second ends **97, 98,** a walled surface **99** disposed between the first and second ends. Fenestrations **94** are molded, laser cut, die cut, or machined in the wall of the tube. ECM material (not shown) can be incorporated into the biodegradable polymer of the injection molded or extruded fenestrated tube, or coated on its surface.

The stent **80** or **90** can be provided as a sterile device that is compressed to a first diameter in the range of about 6 mm to 10 mm and inserted into a reusable delivery tool (not shown) in the operating room immediately before implantation. Once the stent **80** or **90** is deployed, it self-expands outwardly to a variable second diameter conforming to the lumen. The size of the lumen together with the elasticity and circumferential pressure of the surrounding tissues determine the stent's final nominal diameter. The stents' non-compressed, or resting state, diameter, can be in the range of about 12 mm to 18 mm.

A urethral stent can be used to treat urine flow problems, such as those associated with benign prostatic hypertrophy (BPH) or urethral strictures. In this disease the internal lobes of the prostate slowly enlarge and progressively occlude the urethral lumen. A urethral stricture is a circumferential band of fibrous scar tissue which progressively contracts and narrows the urethral lumen. The biodegradable urethral stent with ECM can temporarily restore, or maintain patency of the ureter or the urethra.

In some embodiments, the pelvic implant is in the form of penile implant. Material used to prepare the penile implant can be associated with an ECM material, and the implant can include a degradable material. Penile implants can be useful in treatment of erectile dysfunction (ED). For example, ECM material provided by a penile implant could promote the regeneration of nerves in a treatment area to help improve symptoms of ED.

In another embodiment, the invention provides a biodegradable implant associated with ECM material that is in the form of a pouch. Pouch-type implants could be used to be placed around the bladder to help regenerate nerves to help treat ICS and OAB or within the bladder to help regenerate the lining of the bladder after resection of tumors in the case of bladder cancer.

## Claims

1. An implant configured for treatment of a pelvic disorder, the implant comprising
- a biodegradable polymeric mesh structure, wherein the mesh structure has a plurality of interwoven fibers (12a, 12b, 12c, 12d, 12e), the plurality of interwoven fibers (12a, 12b, 12c, 12d, 12e) including a biodegradable polymer (23), and
- a plurality of extracellular matrix (ECM) particles (21) disposed within at least one fiber of the plurality of interwoven fibers (12a, 12b, 12c, 12d, 12e),
wherein the biodegradable polymer (23) of the at least one fiber (12a, 12b, 12c, 12d, 12e) is configured to operate as a timed-release mechanism for the plurality of ECM particles (21),
wherein the at least one fiber has a coating of ECM particles that are adhered to the outer surface of the fiber by a binder,
wherein the implant is a stent, a urethral sling, a urethral stent, a penile implant or a pouch.

2. The implant of claim 1 which is configured for the treatment of a disorder selected from the group consisting of prolapse, urinary incontinence, fecal incontinence, interstitial cystitis, and urge incontinence.

3. The implant of any one of the previous claims which is configured for the treatment of prolapse or urinary incontinence.

4. The implant of any one of the previous claims, wherein the biodegradable polymer is selected from the group consisting of poly(glycolic acid) (PGA), (PDS), and poly(glycolic lactic acid) PGLA.

5. The implant of any one of the previous claims, where the extracellular matrix particles (21) are powderized ECM particles.

6. The implant of any one of the previous claims, wherein a powderized ECM coating (47) is formed by a process selected from the group consisting of dipping, spraying, and electro deposit.

7. The implant of any one of claims 5 - 6, wherein the powderized ECM (21) is impregnated into the biodegradable polymer (23) using a process selected from the group consisting of swelling of implant material, and vacuum incorporation.

8. The implant of any one of the previous claims which does not include any, or any substantial amount of inert material or non-degradable material.

9. The implant of any one of the previous claims which has 50% (wt.) or greater, 60% (wt.) or greater, 70% (wt.) or greater, 80% (wt.) or greater, 90% (wt.) or greater, or 95% (wt.) or greater, biodegradable polymeric material.

10. A biodegradable urethral stent comprising the implant according to any of claims 1 to 8.

11. The implant according to one of claims 1 to 9 or the stent according to claim 10 configured for treatment of a pelvic disorder, the implant formed from material comprising an extrusion comprising the biodegradable polymeric material (23) and the extracellular matrix particles (21).

## Patentansprüche

1. Implantat, das zur Behandlung einer Beckenerkrankung ausgelegt ist, wobei das Implantat umfasst:
eine biologisch abbaubare polymere Gitterstruktur, wobei die Gitterstruktur mehrere miteinander verwobene Fasern (12a, 12b, 12c, 12d, 12e) aufweist, wobei die mehreren miteinander verwobenen Fasern (12a, 12b, 12c, 12d, 12e) ein biologisch abbaubares Polymer (23) aufweisen, und
mehrere Extrazellulärmatrix(ECM)-Teilchen (21), die in mindestens einer Faser von den mehreren miteinander verwobenen Fasern (12a, 12b, 12c, 12d, 12e) angeordnet sind,
wobei das biologisch abbaubare Polymer (23) der mindestens einen Faser (12a, 12b, 12c, 12d, 12e) dafür ausgelegt ist, als Menchanismus zur zeitvresetzten Freigabe für die mehreren ECM-Teilchen (21) zu wirken,
wobei die mindestens eine Faser eine Beschichtung aus ECM-Teilchen aufweist, die durch ein Bindemittel an der Außenfläche der Faser haften,
wobei das Implantat ein Stent, eine Harnröhrenschlinge, ein Harnröhrenstent, ein Penisimplantat oder ein Beutel ist.

2. Implantat nach Anspruch 1, das für die Behandlung einer Krankheit ausgelegt ist, die ausgewählt ist aus der Gruppe bestehend aus Prolaps, Harninkontinenz, Stuhlinkontinenz, interstitieller Zystitis und Dranginkontinenz.

3. Implantat nach einem der vorangehenden Ansprüche, das für die Behandlung eines Prolapses oder von Harninkontinenz ausgelegt ist.

4. Implantat nach einem der vorangehenden Ansprüche, wobei das biologisch abbaubare Polymer ausgewählt ist aus der Gruppe bestehend aus Poly(glycolsäure) (PGA), (PDS) und Poly(glycol-Milchsäure) PGLA.

5. Implantat nach einem der vorangehenden Ansprüche, wobei die Extrazellulärmatrixteilchen (21) pulverisierte ECM-Teilchen sind.

6. Implantat nach einem der vorangehenden Ansprüche, wobei eine Beschichtung (47) aus pulverisierter ECM anhand eines Prozesses ausgebildet wird, der ausgewählt ist aus der Gruppe bestehend aus Tauchen, Sprühen und Elektro-Abscheiden.

7. Implantat nach einem der Ansprüche 5-6, wobei die pulverisierte ECM (21) anhand eines Prozesses, der ausgewählt ist aus der Gruppe bestehend aus Quellenlassen eines Implantatmaterials und Vakuumeinbringen in das biologisch abbaubare Polymer (23) imprägniert wird.

8. Implantat nach einem der vorangehenden Ansprüche, das inertes Material oder nichtabbaubares Material gar nicht oder in keiner nennenswerten Menge aufweist.

9. Implantat nach einem der vorangehenden Ansprüche, das 50 % (Gew.) oder mehr, 60 % (Gew.) oder mehr, 70 % (Gew.) oder mehr, 80 % (Gew.) oder mehr, 90 % (Gew.) oder mehr oder 95 % (Gew.) oder mehr an biologisch abbaubarem Polymermaterial aufweist.

10. Biologisch abbaubarer Harnröhrenstent, der das Implantat nach einem der Ansprüche 1 bis 8 umfasst.

11. Implantat nach einem der Ansprüche 1 bis 9, oder Stent nach Anspruch 10, ausgelegt zur Behandlung einer Beckenerkrankung, wobei das Implantat aus Material gebildet ist, das eine Extrusion umfasst, die das biologisch abbaubare Polymermaterial (23) und die Extrazellulärmatrixteilchen (21) umfasst.

## Revendications

1. Implant configuré pour le traitement d'un trouble pelvien, l'implant comprenant :
une structure de maillage polymérique biodégradable, dans lequel la structure de maillage comporte une pluralité de fibres entretissées (12a, 12b, 12c, 12d, 12e), les fibres de la pluralité de fibres entretissées (12a, 12b, 12c, 12d, 12e) incluant un polymère biodégradable (23) ; et
une pluralité de particules de matrice extracellulaire (ECM) (21) qui sont disposées à l'intérieur d'au moins une fibre de la pluralité de fibres entretissées (12a, 12b, 12c, 12d, 12e) ; dans lequel :
le polymère biodégradable (23) de l'au moins une fibre (12a, 12b, 12c, 12d, 12e) est configuré de manière à ce qu'il joue le rôle fonctionnel de mécanisme de libération temporisée pour la pluralité de particules d'ECM (21) ; dans lequel :
l'au moins une fibre comporte un revêtement de particules d'ECM qui sont amenées à adhérer sur la surface externe de la fibre au moyen d'un agent de liaison ; et dans lequel :
l'implant est une stent, une attelle urétrale, un stent urétral, un implant pénien ou une poche.

2. Implant selon la revendication 1, lequel est configuré pour le traitement d'un trouble qui est sélectionné parmi le groupe qui est constitué par le prolapsus, l'incontinence urinaire, l'incontinence fécale, la cystite interstitielle et l'incontinence par impériosité.

3. Implant selon l'une quelconque des revendications précédentes, lequel est configuré pour le traitement du prolapsus ou de l'incontinence urinaire.

4. Implant selon l'une quelconque des revendications précédentes, dans lequel le polymère biodégradable est sélectionné parmi le groupe qui est constitué par le poly(acide glycolique) (PGA), le (PDS) et le poly(acide lactique glycolique) (PGLA).

5. Implant selon l'une quelconque des revendications précédentes, dans lequel les particules de matrice extracellulaire (21) sont des particules d'ECM réduite en poudre.

6. Implant selon l'une quelconque des revendications précédentes, dans lequel un revêtement d'ECM réduite en poudre (47) est formé au moyen d'un processus qui est sélectionné parmi le groupe qui est constitué par un trempé, une pulvérisation et un dépôt électrolytique.

7. Implant selon l'une quelconque des revendications 5 et 6, dans lequel l'ECM réduite en poudre (21) est imprégnée à l'intérieur du polymère biodégradable (23) en utilisant un processus qui est sélectionné parmi le groupe qui est constitué par un gonflement du matériau d'implant et une incorporation sous vide.

8. Implant selon l'une quelconque des revendications précédentes, lequel n'inclut ni un matériau inerte, ni un matériau non dégradable, ni une quantité substantielle de l'un de ces matériaux.

9. Implant selon l'une quelconque des revendications précédentes, lequel comporte 50 % (en poids) ou plus, 60 % (en poids) ou plus, 70 % (en poids) ou plus, 80 % (en poids) ou plus, 90 % (en poids) ou plus ou 95 % (en poids) ou plus de matériau polymérique biodégradable.

10. Stent urétral biodégradable comprenant l'implant selon l'une quelconque des revendications 1 à 8.

11. Implant selon l'une des revendications 1 à 9 ou stent selon la revendication 10 configuré pour le traitement d'un trouble pelvien, l'implant étant formé à partir d'un matériau qui comprend une extrusion qui comprend le matériau polymérique biodégradable (23) et les particules de matrice extracellulaire (21).
